# EUROPEAN PATENT SPECIFICATION

(11) **EP 4 476 317 B1**
(45) Date of publication and mention of the grant of the patent: **05.11.2025**
(21) Application number: 23718379.3
(22) Date of filing: 06.02.2023
(51) Int. Cl.: C12M 1/26, F04B 49/00, F04B 49/02, F04B 23/04, F04B 17/06

(54) **DEPTH FILTER AND VIRAL FILTER (DF/VF) CART FOR BATCH AND CONTINUOUS PROCESSING**
TIEFENFILTER UND VIRENFILTER (DF/VF)-WAGEN ZUR BATCH- UND KONTINUIERLICHEN VERARBEITUNG
CHARIOT À FILTRE EN PROFONDEUR ET FILTRE ANTIVIRAL (DF/VF) POUR TRAITEMENT DISCONTINU ET CONTINU

(30) Priority: 10.02.2022 US 202263308878 P
(43) Date of publication of application: 18.12.2024
(73) Proprietor: Just-Evotec Biologics, Inc., Seattle, WA 98109 (US)
(72) Inventor: GEFROH, Eva Fan, Newcastle, WA 98056 (US); CABLE, Cristopher James, Bellevue, WA 98005 (US); WANEK, Timothy Joel, Seattle, WA 98117 (US)
(74) Representative: Latscha Schöllhorn Partner AG
(86) International application number: PCT/US2023/012395
(87) International publication number: WO 2023/154245

(56) References cited:
- EP-A1- 2 415 856
- US-A1- 2019 083 910
- US-A1- 2019 374 874

## Description

### BACKGROUND OF THE INVENTION

### 1. Field of the Invention

This invention relates to automated manufacturing facilities, devices, and processes for the industrial production of proteins.

### 2. Discussion of the Related Art

The biopharmaceutical industry is undergoing major changes, prompted in part by the surge in approvals of new biotherapeutics, higher protein expression rates and increased pressure from the biosimilars market. (Levine et al., Efficient, flexible facilities for the 21st century, BioProcess International 10(11):20-30 (2012)).

A surge in the pharmaceutical market share of biologics (from 11% in 2002 to around 20% in 2017), coupled with the need for affordable medicine access in developing regions of the world requires the development of fast, sustainable and cost-effective manufacturing methods. (Walsh, Biopharmaceutical benchmarks 2014, Nature biotechnology 32(10):992-1002 (2014)).

Biologics manufacturing technology alternatives to traditional batch processing platforms to capitalize on key advantages such as higher throughput, operational flexibility and cost savings, as well as footprint reduction reduced environmental impact. Bioprocessing plants designed to contain a continuous manufacturing process with integrated upstream and downstream unit operations, allow for rapid facility turnaround, product and capacity flexibility, and lower costs of manufacturing compared to batch culture processing. (See, e.g., Farid et al., Evaluating the economic and operational feasibility of continuous processes for monoclonal antibodies, Continuous Processing in Pharmaceutical Manufacturing pp. 433-456 (2015); Kelley, Industrialization of mAb production technology: the bioprocessing industry at a crossroads, mAbs 1(5):443-452 (2009); Croughan et al., The future of industrial bioprocessing: Batch or continuous?, Biotechnology and Bioengineering 112:648-651 (2015); Pollock et al., Fed-batch and perfusion culture processes: Economic, environmental, and operational feasibility under uncertainty, Biotechnology and Bioengineering 110(1):206-219 (2013)).

The advent of continuous perfusion technologies has supported greater progress in connecting the upstream process equipment in order to operate in a continuous mode. This processing strategy has been valuable for several companies over the past 25 years, helping them to overcome stability problems associated with their products. (Konstantinov et al., White paper on continuous bioprocessing, Journal of Pharmaceutical Sciences 104(3):813-820 (2015)).

Modem cell lines and media have been engineered to target higher cell densities, especially when contrasted with fed-batch processing, with some cultures achieving viable cell densities greater than 100 million cells/mL. (Clincke et al., Very high density of chinese hamster ovary cells in perfusion by alternating tangential flow or tangential flow filtration in wave bioreactor™-part ii: Applications for antibody production and cryopreservation, Biotechnology Progress 29(3):768-777 (2013)). As a result of this, there has been a shift in the typical biologics manufacturing facility bottleneck from the production bioreactors (upstream processes) to the purification trains (downstream processes), and in particular the chromatography columns due to their dimensional limitations. Purifying large product batch sizes generated by the rising quantities of protein from current production cell lines is not a trivial challenge. (Chon et al., Advances in the production and downstream processing of antibodies, New Biotechnology 28(5):458-463 (2011)). Thus, the problem of integration of upstream biologics manufacturing processes with downstream processes, continues to trouble the biologics manufacturing industry.

Warikoo *et al.* reported the integration of a continuous capture chromatography step downstream of the production bioreactor, resulting in column size and buffer utilization reductions. (Warikoo et al., Integrated continuous production of recombinant therapeutic proteins, Biotechnology and Bioengineering 109(12):3018-3029.(2012)).

Godawat *et al.* have demonstrated that end-to-end continuous bioprocessing is feasible, but still faces several challenges, including developing robust viral clearance and automation strategies that ensure high product quality. (Godawat et al., End-to-end integrated fully continuous production of recombinant monoclonal antibodies, Journal of Biotechnology 213:13-19 (2015))

Vandiver *et al.* further developed flexible biologics manufacturing technology with the capability of varied production modes: batch, fed-batch culture, intensified fed-batch culture, semicontinuous, or continuous end-to-end processes. (Vandiver et al., Automated Biomanufacturing Systems, Facilities, and Processes, WO2020/168315A1).

Installations and systems, including filter carts, previously known in the art have been inadequate for modem biologics production lines that employ flexible configurations. (See, e.g., Lu, et al., Perfusion Bioreactor With Filtration Systems, US2019/338238A1; Born et al., Continuous Separator Bypass Systems and Methods of Use Thereof, US2020/384380A1; Reinbigler et al., Installation for Treating a Biological Liquid, US2012/0248025A1). Weissenbach, et al. (Millipore Corp.) disclosed a Pump cart for a biological liquid treatment installation in EP2415856A1.

Accordingly, there is a need for a dual-path depth filter and viral filter (DF/VF) cart with the capacity to meet the requirements for efficient and flexible batch and/or continuous biologics manufacturing processes, with interchangeability, and over extended production periods. This the present invention provides.

### SUMMARY OF THE INVENTION

The present invention involves a portable automated single-use filter cart system for interchangeable continuous or batch biologics manufacturing.

The inventive portable automated single-use filter cart system includes:
(a) a portable cart having a perimeter;
(b) a valved product inlet within the perimeter of the cart for receiving an aqueous product-containing fluid from an upstream unit operation outside the perimeter of the portable cart, wherein the upstream unit operation can be fluidly connected to the product inlet by a sealable connector and a connecting line, wherein the product inlet is connected via a connecting line capable of fluidly conveying the aqueous product-containing fluid downstream to a first pump or, interchangeably, to a second pump, wherein the first pump is adapted for low flow rate operation, and the second pump is adapted for high flow rate operation, and wherein the first pump and the second pump are each configured to impel the flow of the aqueous product-containing fluid to a first filtration path or alternately to a second filtration path; wherein the first filtration path and the second filtration path each comprises:
   (i) a plurality of filters downstream from the first pump, wherein the plurality of filters is fluidly connected in sequence along the filtration path; and, optionally, outside the perimeter of the cart, one or more portable accessory filtration module(s) fluidly connected to the filtration path and capable of receiving aqueous product-containing fluid therefrom, and capable of returning filtered aqueous product-containing fluid thereto;
   (ii) a plurality of sensors capable of continuously measuring flow rate, flow volume, and/or pressure at one or more locations along the filtration path and transmitting measurement signals to a controller; and
   (iii) a connecting line capable of fluidly conveying filtered aqueous product-containing fluid to a downstream product outlet port;

The inventive portable automated single-use filter cart system also includes:
(c) one or more valved buffer inlets within the perimeter of the cart and upstream from the first filtration path and the second filtration path, and each buffer inlet fluidly connected by a connecting line capable of fluidly conveying a desired aqueous fluid received from a reservoir or an upstream unit operation, for injection into the first or into the second filtration path by operation of the first pump or the second pump and the valved buffer inlets, in response to instruction signals from the controller; and
(d) the controller is configured and programmable to automatically switch the flow of product-containing fluid from the first filtration path to the second filtration path in response to a predetermined measurement signal received from the sensor(s) of the first filtration path, or after a predetermined period, wherein the controller is further configured to automatically switch the flow of aqueous product-containing fluid from the second filtration path to the first filtration path in response to a predetermined measurement signal received from the sensor(s) of the second filtration path, and wherein the controller is further configured to issue instruction signals: (i) activating the first pump during a desired continuous biologics manufacturing process, or interchangeably, activating the second pump during a desired batch biologics manufacturing process; (ii) activating the second pump during the desired continuous biologics manufacturing process to flush buffer or water through the first filtration pathway while the flow of aqueous product-containing fluid is through the second filtration pathway, or interchangeably, to flush buffer or water through the second filtration pathway while the flow of aqueous product-containing fluid is through the first filtration pathway; and (iii) controlling the valved product inlet and the one or more valved buffer inlets.

One benefit of the present invention is that during a desired continuous biologics manufacturing process, the inventive portable automated single-use DF/VF filter cart supports switching between the first filtration pathway and the second filtration pathway an indefinite number of times, as required by the process run, even if some manual intervention may be required to replace filters, as needed.

The foregoing summary is not intended to define every aspect of the invention, and additional aspects are described in other sections, such as the Detailed Description of Embodiments. The entire document is intended to be related as a unified disclosure, and it should be understood that all combinations of features described herein are contemplated, even if the combination of features is not found together in the same sentence, or paragraph, or section of this document. For example, certain aspects of the invention that are described as a genus, and it should be understood that every member of a genus is, individually, an aspect of the invention.

### BRIEF DESCRIPTION OF THE DRAWINGS

Figure 1 shows an embodiment of the portable automated single-use filter cart system.
Figure 2 shows the embodiment of Figure 1 from a different perspective.
Figure 3 shows another embodiment involving "off-skid" filtration modules. This embodiment of the portable automated single-use filter cart is shown with depth filters (326a) contained in housings or holders outside of the perimeter of the cart in two portable accessory filtration modules, one for each of the first filtration path and the second filtration path.
Figure 4 shows a schematic representation of the design of the inventive portable automated single-use DFVF filter cart system that can operate either a depth filter (426a) or a viral filter (426b) in either batch or continuous mode.
Figure 5 shows depth filter load inlet pressure and load turbidity over time in a representative run with depth filtration, as described in Example 1 herein.
Figure 6 shows viral filter load flow rate and differential pressure over time in a representative run with viral filtration, as described in Example 2 herein.

### DETAILED DESCRIPTION OF EMBODIMENTS

The section headings used herein are for organizational purposes only and are not to be construed as limiting the subject matter described.

### Definitions

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular. Thus, as used in this specification and the appended claims, the singular forms "a", "an" and "the" include plural referents unless the context clearly indicates otherwise. For example, reference to "a protein" includes a plurality of proteins; reference to "a cell" includes populations of a plurality of cells.

The present invention involves a portable automated single-use filter cart system for interchangeable continuous or batch biologics manufacturing. The inventive filter cart can perform under aseptic operational conditions and can be integrated into an automation-controlled and regulated biologics manufacturing process, if desired, whether the manufacturing process is a batch process, a semi-continuous process, or a continuous process. The inventive filter cart system can function interchangeably in any of these process modes, and can be placed between different unit operations, as needed in a particular manufacturing process.

The term "integrated," in connection with a process for manufacturing a purified protein or other biologic of interest (e.g., but not limited to, a protein drug substance), means that one or more upstream steps and/or downstream steps in the manufacturing process are performed under common or coordinated control, based on programmed commands as modified by current sensory feedback of defined parameters in relation to set-points or flow rates. The term "coordinated" means that two or more operations, steps, processes, components, or systems, are controlled, regulated, or scheduled in a relationship that will ensure efficiency or harmony of their functioning toward a single purpose.

In a typical biologics manufacturing process, after harvesting the cell culture product-containing fluid comprising a protein or other biologic product of interest (e.g., an antibody), the product can be further purified from the cell-free supernatant fraction. Typically, the purification of proteins is usually accomplished by an optional series of chromatographic steps such as anion exchange chromatography, cation exchange chromatography, affinity chromatography (using Protein A or Protein G or Protein L as an affinity ligand), hydrophobic interaction chromatography, hydroxy apatite chromatography and size exclusion chromatography. Further, the purification process may comprise one or more depth filtration, viral filtration, ultra-, nano- or diafiltration steps, and/or, optionally, an acidic viral inactivation step. Other optional known techniques for protein purification such as ethanol precipitation, Reverse Phase HPLC, chromatofocusing, SDS-PAGE, and ammonium sulfate precipitation are also possible depending on the biologic to be recovered.

In general, "upstream" process steps include, but are not limited to, e.g., culturing product-producing cells (e.g., but not limited to recombinant host cells) in a bioreactor (e.g., a perfusion bioreactor); removing cells from a permeate; and fluidly feeding volumes of cell-free permeate from the bioreactor(s), optionally, whether into an intervening surge vessel, or directly into a downstream process step. "Downstream" process steps can include, but are not limited to, e.g., product capture and purification in at least one or more chromatography systems; a viral inactivation system, such as a low pH or detergent viral inactivation system and, if needed (e.g., in low pH viral inactivation system embodiments), a neutralization system; a depth filtration system; a viral filtration system; and/or an ultrafiltration/diafiltration system. In a given manufacturing process, the order or details of these process steps may vary. However, a first manufacturing process step, or unit operation, is also said to be "upstream" in relation to a second unit operation that follows (directly or indirectly) the first unit operation in the manufacturing process; and the second unit operation is said to be "downstream" in relation to the first unit operation that (directly or indirectly) precedes the second unit operation. Within a unit operation, e.g., within the portable automated single-use filter cart system of the invention, "upstream" refers to a first location or function within the system that precedes (based on the direction of flow of the product-containing fluid through the system) a second location or function, which is referred to as being "downstream" from the first location or function within the system.

A "continuous" format or mode of a manufacturing process or system means a processing modality wherein a perfusion bioreactor is fluidly connected to a continuous capture chromatography step (e.g., processing by a first chromatography system) in an uninterrupted flow coming from the bioreactor (directly or indirectly via intervening unit operations) to the first chromatography system, which is followed by, and fluidly connected in an uninterrupted flow to, a downstream viral inactivation step, and optionally, in an uninterrupted flow to depth filtration. Further downstream product purification steps (e.g., a second chromatography system, an optional third chromatography system, viral filtration, and processing by ultrafiltration/diafiltration) are fluidly connected, all in an uninterrupted flow to the afore-mentioned upstream processing steps and successively to each other, with optional intervening surge vessels.

A "semi-continuous" format or mode of a manufacturing process means a processing modality wherein a perfusion bioreactor is fluidly connected to a continuous capture chromatography step (e.g., processing by a first chromatography system) in an uninterrupted flow, and to processing by another step, such as but not limited to, a viral inactivation system, and optionally, in an uninterrupted flow to depth filtration, and storage of virally inactivated product pool in a holding vessel, before further processing. Temporary storage of the product pool in the holding vessel is subsequently followed by one or more batch downstream processing step(s), which step(s) can be successively fluidly connected to each other in an uninterrupted flow, e.g., a second chromatography system, an optional third chromatography system, and processing by ultrafiltration/diafiltration, with optional intervening surge vessels or holding vessels (i.e., holding vessels if there are two or more batch steps or operations), as the case may be.

A "perfusion bioreactor" is a bioreactor for culturing cells in which equivalent volumes of culture medium can be added and removed from the reactor while the cells are retained in the bioreactor. A perfusion bioreactor includes a bioreactor and an operably attached perfusion system, which provides a steady source of fresh nutrient medium and removal of cell waste products. Typically, the bioreactor and the perfusion system of the perfusion bioreactor can be separate mechanical units that operate in coordination. Numerous commercially available examples include, but are not limited to, a variety of Xcellerex^{®} brand single-use bioreactors (SUBs; GE Healthcare Life Sciences) and KrosFlo^{®} brand perfusion flow-path assemblies and systems (Spectrum; Repligen), which bioreactors and perfusion systems can be suitably combined into a perfusion bioreactor by the skilled practitioner. Alternatively, the bioreactor and the perfusion system can be assembled into a single mechanical unit, for example, but not limited to, a 3D Biotek brand perfusion bioreactor (Sigma-Aldrich). Secreted protein products in the bioreactor can be continuously harvested by microfiltration during the process of removing medium via the perfusion system, the protein of interest thus being isolated in a microfilter permeate exiting the perfusion system.

A step of a manufacturing process, or a system within an automated manufacturing facility, is performed "fluidly," or is "fluidly connected" to, or "fluidly receives" material from, another step of the manufacturing process or from another system, when material containing the protein of interest flows by pipe, tubing, or other closed conduit between steps or systems without manual loading or unloading. Such pipe, tubing, or other closed conduit between steps or systems is considered a "connecting line," if it is capable of fluidly conveying the aqueous product-containing fluid downstream within a system or between systems or unit operations. The connecting line can be made of any suitable nonreactive, non-porous material, such as, but not limited to silicone rubber, Teflon^{®}, stainless steel, or the like. Aseptic connectors are useful to join connecting lines to other components, as the skilled person is aware.

A step of a manufacturing process or a system within an automated manufacturing facility is commonly called a "unit operation." A "production line" or "production process line" (used interchangeably herein) is a series of unit operations involved in the production of a product, such as but limited to, a biologic, e.g., a protein of interest, a drug substance, or a drug product.

A "batch unit" or "skid" is a unit operation. Typical examples can include: a single-use bioreactor(s), a perfusion system, a first chromatography system, an optional second chromatography system, an optional third chromatography system, and/or an ultrafiltration/diafiltration system, each of which can be, but are not necessarily, configured as "skids." A viral inactivation system, and if needed a neutralization system, can also be a "skid." Batch units are configured to communicate (e.g., by hard wiring, fiber optic cables, or wireless connections) with a Process Control System (PCS) or Supervisory Control and Data Acquisition (SCADA) system. The portable automated single-use filter cart system of the present invention can also be configured as a "skid," within a manufacturing process. A "non-batch unit" is equipment that supports one or more batch units. Non-batch units are configured to communicate (e.g., by hard-wiring, fiber optic cables, or wireless connection) with the PCS or SCADA. Additionally, a dongle and/or a Profibus device, or similar digital information storage device and electronic hardware connector(s), may be attached to the non-batch unit to identify its location (association with the batch unit) and role (e.g., Feed Tank, Collection Tank, Filter Bank, etc.). For convenience and flexibility, filter banks, heat exchangers, surge vessels, feed tanks, reservoirs, holding vessels, collection vessels or collection tanks (e.g., an elution collection vessel), and portables mixers and other mixing vessels, when optionally present, are typically configured as non-batch units, although in some embodiments, unit operations such as these can also be included in a "skid," involving control via hard-wiring, fiber optic cables, or wireless connection.

The terms "automated," "automation-controlled," or "automatically," are used interchangeably, in connection with a manufacturing process or facility, and refer to computer-control of the implementation or performance of one or more process steps or the operation of a component or system of a manufacturing facility, optionally, with attendant feed-back regulation of the process step or operation. Typically, a computerized controller receives digital signals from detectors of the physical or chemical parameter to be controlled and issues responsive digital instructions to a system or subsystem.

The terms "single-use" or "single use," used herein interchangeably, means that a particular aseptic production line component, i.e., an aseptic piece of equipment, for example a component used in the inventive portable automated single-use filter cart system is constructed or configured to be employed for a single production run (but may be re-used if quality and aseptic sanitation can be assured for multiple runs). The single-use component can then be disposed of and replaced for subsequent production runs by another single-use component of the same or modified configuration without the need for cleaning and sanitization of the component between production runs. Such single-use components can be constructed or obtained commercially. Examples of single-use components that can be employed in the present invention include, but are not limited to, a depth filter, a viral filter, a pre-filter, a sterilizing grade filter, or any of the connectors, connecting lines or valves, containers or bags. Single-use depth filters, single-use viral filters, single-use pre-filters, and single-use sterilizing grade filters are widely available commercially, e.g.: single-use filter assembly systems containing filters (various membrane and pore sizes from MilliporeSigma or Sartorius Stedim Biotech); single-use viral filtration systems, such as Allegro^{™} MVP Single Use System Manifolds (Pall Biotech); Mobius^{®} FlexReady for Viral Filtration (MilliporeSigma); FlexAct^{®} for Viral Filtration (Sartorius), Planova^{™} Single-Use Virus Filtration (SU-VFS; Asahi Kasei Bioprocess America, Inc.), or Viresolve^{®} Pro Virus Filtration (MilliporeSigma). Single-use connecting lines of various dimensions, lengths, and configurations using disposable silicone and/or c-flex type tubing are commercially available from Thermo Fisher Scientific (ASI), Advantapure, Pall, Colder, GE Healthcare Life Sciences, and Sartorius Stedim Biotech; a system of connecting lines is also known as a "tubing manifold." Single-use aseptic connectors can be obtained from various manufacturers including AseptiQuik^{®} connectors (Colder Products Company), Kleenpak^{®} Presto Sterile Connector (Pall Biotech); Lynx^{®} ST Connector (MilliporeSigma).

The term "switching," or "switch," used herein interchangeably, with respect to the first filtration path and the second filtration path, means to change the direction of flow of the aqueous product-containing fluid from the first filtration path to the second filtration path, or vice versa. Such switching can be under the automatic control and regulation of a computer, i.e., the controller (140, 240, 440), and mechanism(s), e.g., valves (110, 210, 310, 410) and pumps (116a, 116b, 216a, 316a, 316b, 416a, 416b), which optionally can be governed by an external control system, e.g., an automated production facility control system. Switching can be manually controlled and regulated, however, "automatically switching" means that the switch in flow direction does not require manual input on the operator's part, but is controlled instead by the controller (140, 240, 440), which performs the switch in flow direction under predetermined criteria or set-points.

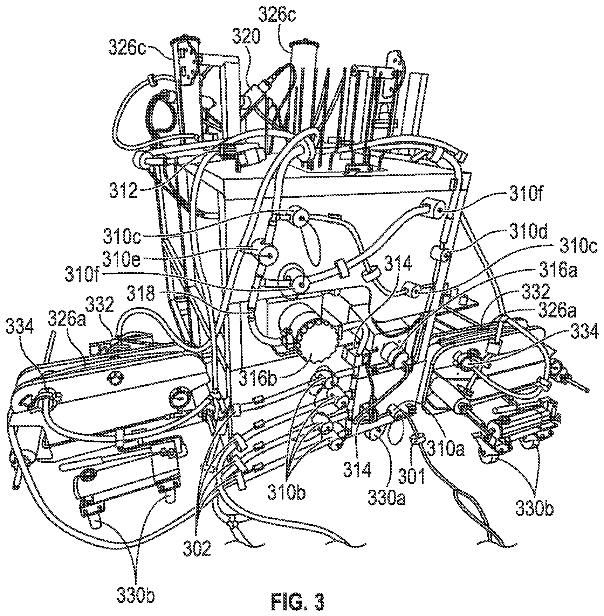

An "accessory filtration module" is a filtration unit outside of the perimeter of the filter cart which is fluidly connected to and capable of being incorporated into either the first filtration path or the second filtration path. (See, e.g., the embodiment of Figure 3).

The term "sterile" or "aseptic," as used herein, refers to a substance that is free, or essentially free, of microbial and/or viral contamination. In this respect a "contaminant" means a material that is different from the desired components in a preparation, such as a cell culture medium, a component of a cell culture medium, or an aqueous product-containing fluid, or any other preparation. In the context of "sterile filtration", the term sterile filtration is a functional description that a preparation is filtered through a sterile filter (with a pore size of 0.2 µm or less) to remove bacterial and/or mycoplasma contaminants.

"Batch filtration," otherwise known as "batch wise filtration" or filtration done in batch mode, refers herein to a process wherein a specific total amount or volume of a preparation, such as a cell culture medium or at least a component of a cell culture medium, or an aqueous product-containing fluid, or any other preparation, is filtered through a filter, such as, but not limited to, a depth filter or a virus filter in one batch dependent on the capacity of the filter and wherein the filtration process is finalized before the filtrate is directed or fed to the process in which it is used or consumed.

The term "continuous filtration" or "online filtration" or "in line filtration" refers to a filtration process, wherein the specific total amount or volume of a preparation, being a cell culture medium or at least a component of a cell culture medium, or an aqueous product-containing fluid, or any other preparation, is filtered through a filter, such as, but not limited to, a depth filter or a virus filter, continuously dependent on the capacity of the filter and wherein the filtration process is still going on when the filtrate is already directed or fed to the process in which it is used or consumed.

The "cell culture supernatant" is the extracellular medium in which the product-producing cells are cultured. This medium is not to be confused with feed medium that may be added to the culture after inoculation of the cells into the cell culture medium and cell growth has been commenced. A "cell culture" means the cell culture supernatant and the product-producing cells cultured therein. Conventionally, mammalian product-producing cells are cultured at 37°C ± 1°C.

"Culturing at" or "maintaining at" a given temperature or other condition parameter (e.g., pH, oxygenation, CO₂ concentration, pressure, etc.), means that the process control systems are set to the intended target temperature within a unit operation; the portable automated single-use filter cart system of the present invention can include such automated regulation to maintain desired conditions. The culture or unit operation conditions, such as temperature (typically, but not necessarily, about 37°C), pH, oxygenation, and the like, are maintained at the desired set points by digital control units (DCUs) and sensory monitors that are available commercially, or can be constructed by the skilled artisan. Digital control units (DCU) capable of controlling and monitoring the conditions of unit operations within a manufacturing process are available commercially, made by companies such as B. Braun, New Brunswick, or Sartorius.

For any given condition set-point, slight variations may occur during process operations. Suitable monitoring equipment and appropriate alternatives are commercially available or can be constructed by the skilled artisan.

The term "buffer" or "buffered solution" refers to solutions which resist changes in pH by the action of its conjugate acid-base range. Examples of useful buffers that control pH at ranges of about pH 4 to about pH 8 include phosphate, bicarbonate, acetate, MES, citrate, Tris, bis-tris, histidine, arginine, succinate, citrate, glutamate, and lactate, or a combination of two or more of these, or other mineral acid or organic acid buffers. Salts containing sodium, ammonium, and potassium cations are often used in making a buffered solution.

The term "loading buffer" or "equilibration buffer" refers to the buffer, and salt or salts, which is mixed with a product-containing preparation (e.g., a batch or perfusion culture supernatant or filtrate, or an eluant pool containing the biologic product interest, e.g., an antibody) for loading the protein preparation onto an affinity chromatography matrix, e.g., Protein A- or Protein G- conjugated matrix or a specific target ligand-conjugated affinity chromatography matrix, as the case may be, or for loading onto a depth filter or viral filter. This buffer is also used to equilibrate the applicable matrix or filter before loading, and to wash after loading the biologic product.

### The Cart

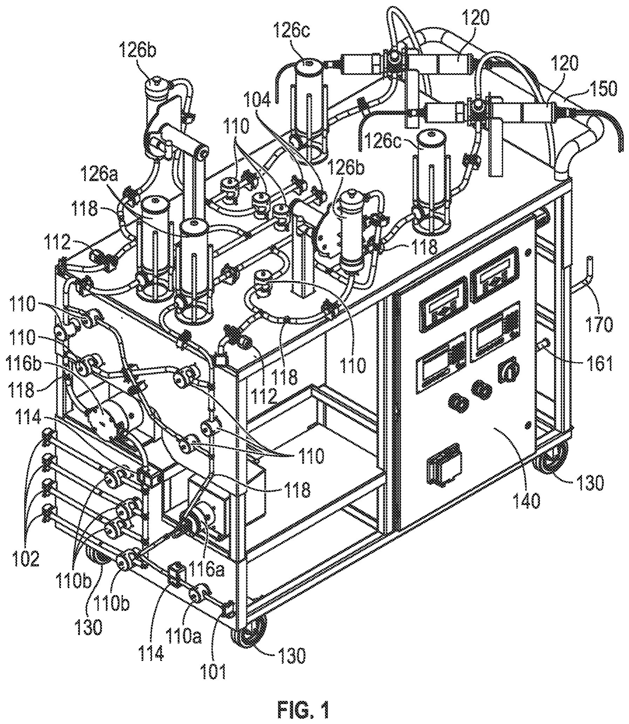

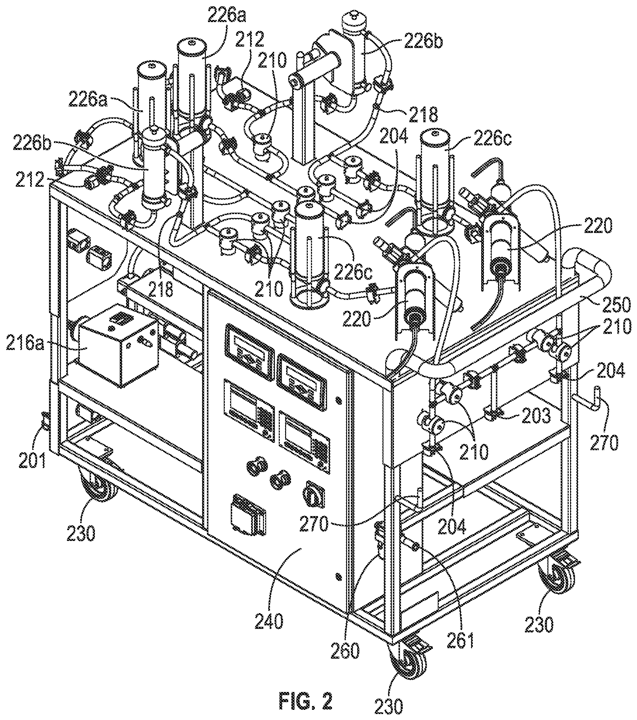

The portable automated single-use filter cart system of the invention includes a cart which has a perimeter or boundary, and which includes a frame, a top, a base, and preferably, wheels, spheres, sleds, casters, or other suitable device at the base to enhance the portability of the cart. (See, e.g., Figure 1 at (130), Figure 2 at (230), and Figure 3 at (330a)). Wheels, spheres, sleds, casters, or other suitable devices to enable, or enhance, portability can also be included on the base of an accessory filtration module. (see, e.g., Figure 3 at (330b)). The frame of the cart, the top of the cart, the base, and any optional shelves, trays, drawers, hooks, clips, snaps, plumbing, included on the cart are considered as being with the perimeter of the cart. In different embodiments, the shape of the cart can be varied as convenient for the desired application or purpose within the production line, although a generally rectangular or cubical three-dimensional shape is often most convenient for many applications. (See, e.g., Figure 1, Figure 2, and Figure 3). The cart can be constructed of any suitable material(s), such as but not limited to stainless steel, aluminum, titanium, metal alloys, plastics, or any other suitably strong, durable, and cleanable and/or sanitizable material or combination of materials. In one embodiment, 316L stainless steel is used to construct the cart, due to this material's resistance to corrosion and its cleanability. The size of the cart can vary, as desired for a particular purpose. In some typical embodiments, the dimensions of the cart perimeter are about 65 inches x 32 inches x 45 inches (Length x Width x Height).

In some embodiments, the controller is situated within the perimeter of the cart, e.g., within the boundaries of the frame (see, e.g., Figure 1 at 140) and Figure 2 at 240), or on top of the cart. In other embodiments, the controller can be outside of the perimeter of the cart itself, so long as the controller is able to communicate with the pumps, the valves, and the sensors on the filter cart, i.e., that the controller can receive measurement signals transmitted from the sensors, and the controller can transmit instructions to all the pumps and valves. Transmission and/or reception of such measurement signals or instruction signals, as the case may be, can be via electrical cables, wires, fiber optic cables, or can be via infrared or radio wave transmissions, and/or any other suitable medium.

In some embodiments, hooks (e.g., in Figure 1 at (170) and Figure 2 at (270)), loops, clips, and/or trays are included in any convenient locations on the cart. Such hooks, loops, and/or clips are for cable management for power cords, signal transmission cables or wires, and/or tubing or plumbing management or support. For example, trays or hooks for hold tubing or cables or wires that extend beyond the perimeter of the system, to enhance and maintain tidiness. In some embodiments there are some small plastic clips attached to the front and back face of the cart (or other vertical cart sections) to hold the tubing in place.

### Pumps

The pumps (116a, 116b, 216a, 316b, 416a, 416b) can be peristaltic or positive displacement. Pumpheads can be single-use and product-contacting pumpheads that can be replaced from run to run or traditional, non-product-contacting pumpheads, such as but not limited to, typical peristaltic pumps, which are reusable. For example, in some embodiments the second pump (for high flow rate operation) is a Quattroflow 1200SU HT, single-use quaternary diaphragm pump with a flow rate range of about 6 to 1200 L/hour; the first pump (for low flow rate operation) is a Quattroflow^{™} 150SU pump motor retrofitted with Quattroflow^{™} 30SU pump head with flow rate range of about 0.06 to about 30 L/hour. Other pumps capable of operation in the afore-mentioned flow rate ranges are also useful for the inventive DF/VF filter cart, and many are commercially available.

### Valves

The automated valves of the inventive portable automated single-use filter cart system can be of any suitable type to regulate fluid flow through the connecting lines. In some embodiments the automated valves are robotically regulated mechanical valves, pneumatic valves, hydraulic valves, diaphragm valves, ball valve, gate valves, needle valves, bellows valves, or globe valves. If a source of compressed air is available, pneumatic valves are particularly useful. A pinch valve type, which has a block that drops down onto the tubing to pinch the tubing of a connecting line shut is a useful embodiment (e.g., pinch valves manufactured by Aquasyn LLC or Acro Associates, a wholly-owned subsidiary of Bimba Manufacturing Corporation). A variety of pinch valves are useful, including, but not limited to, overmolded tubing assemblies to reduce dead volume in the tubing. There are other useful options that include multiplexing valves together, e.g., in 3-way or 4-way configurations. The skilled person knows how to select the optimal valves for a given embodiment, in view of the desired level of simplicity, cost effectiveness, and the nature of the tubing assembly chosen, with a view to minimizing valve footprint and dead volumes.

### Controllers and Automated Production Processes.

The controller (140, 240, 440) of the portable automated single-use filter cart system of the present invention can be configured and programmable to automatically switch the filtration path of the filter cart system independently, based on pre-programmed instructions, set-points, and measurement signals transmitted by the cart's own pressure sensors (118, 218, 318, 418), flowmeters (114, 314, 414), and other analytical sensors (120, 220, 320, 420) monitoring UV (420a), conductivity and temperature (420b, 420c), and pH (420d). The controller (140, 240, 440) is configured and programmable to automatically switch the flow of aqueous product-containing fluid from the first filtration path to the second filtration path or, conversely, from the second filtration path to the first filtration path, in response to a predetermined measurement signal received from the sensor(s) of the second filtration path, and the controller is further configured and programmable to issue instruction signals (i) activating the first pump during a desired continuous biologics manufacturing process, or interchangeably, activating the second pump during a desired batch biologics manufacturing process; (ii) activating the second pump during the desired continuous biologics manufacturing process to flush buffer or water through the first filtration pathway while the flow of aqueous product-containing fluid is through the second filtration pathway, or interchangeably, to flush buffer or water through the second filtration pathway while the flow of aqueous product-containing fluid is through the first filtration pathway; and (iii) controlling the valved product inlet and the one or more valved buffer inlets.

However, in some embodiments, the controller (140, 240, 440) can also be configured and programmable to interact with an overarching production facility control system. In general, production facility control systems can be used to control automated production lines that have flexible configurations, including individual unit operations, such as the portable automated single-use filter cart system of the present invention. That is, the production facility control system can be designed to accommodate multiple configurations that utilize portable equipment that can be coupled to other components of the production line. The production line can include one or more skids that include original manufacturer's equipment, such as a single-use bioreactor system, a perfusion system, or a continuous chromatography system. The skids can also include flow control devices, such as pumps. Additionally, the skids can include one or more communication interfaces that enable the physical coupling of portable pieces of equipment to the skid. The physical coupling between the portable pieces of equipment and the skid can be achieved using electrical cabling. The electrical cabling can be configured to accommodate serial, bus, or ethernet communications. In some embodiments, the electrical cabling can be Recommended Standard 232 (RS-232) cabling.

The portable pieces of equipment, such as the portable automated single-use filter cart system of the present invention, can include, or can otherwise be coupled to, a network gateway hardware device that enables communication between the skid and the production facility control system. In addition, at least some of the skids can be logically configured to be coupled to various pieces of portable equipment. In this way, the pieces of portable equipment can be physically connected to a particular skid based on the configuration of a particular production line and the skids can be configured to operate in different configurations based on the different pieces of equipment coupled to the skid.

The location of the portable automated single-use filter cart system of the present invention for Depth Filtration or Viral Filtration operations in the production line can be assigned and/or identified by the production facility control system. Furthermore, specific sequence logic for specific Depth Filtration or Viral Filtration processes are also facilitated by the production facility control system in the form of automated recipes or methods conforming to ISA-88 and ISA-95 (and/or other applicable) standards to control one or more control modules, flags, and/or status identifiers for a depth filter and/or viral filter, and execute the one or more control modules while the filter cart. The production facility control system can monitor various process conditions and signals such as pressure or flow rate.

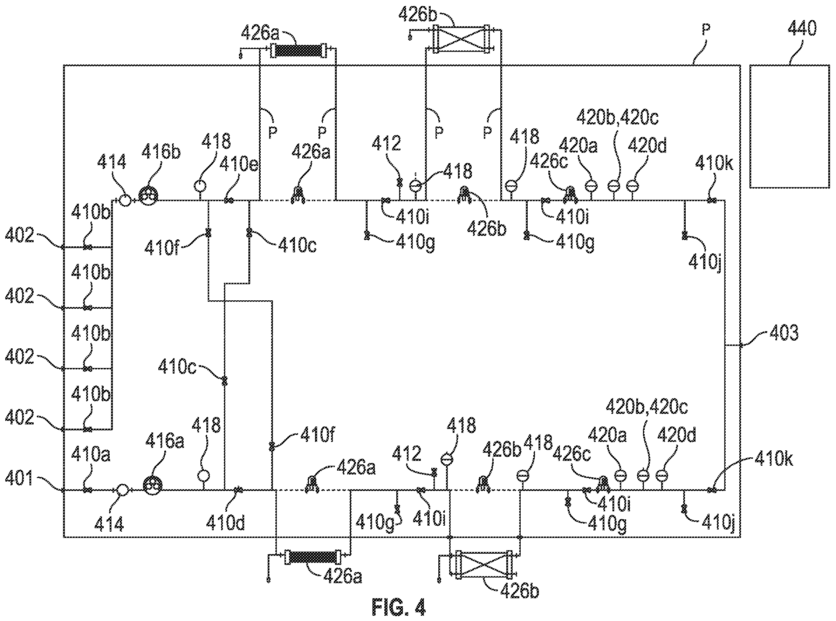

For example, pressure within the filter assemblies of the filter cart can be monitored, based on pressure values obtained from pressure sensors (118, 218, 318, 418) included in the filter cart assemblies and relayed by the filter cart controller (140, 240, 440). The production facility control system can determine that the pressure within a first filter assembly through which material is flowing has reached at least a threshold level. The threshold level of pressure can indicate that a filter included in the first assembly needs to be replaced due to a decrease in the amount of material that can be processed by the filter, and an instruction signal and/or alarm signal can be issued. The production facility control system can then send a signal to the controller (140, 240, 440) to switch the flow of aqueous product-containing fluid from the first filtration path to the second filtration path, by operation of the valves as described in greater detail in the schematic illustration of Figure 4. If desired, for example, over a long production run, the filters included in the first and/or second filtration path can be replaced. Similarly, the controller can be programmed to respond to other preset parameters (e.g., time duration, volume throughput), or trigger conditions measured by the analytical sensors (120, 220, 320, 420) that prompt the controller to activate the flow of the aqueous product-containing fluid to be switched from one flow path to the other, and/or to respond to operator prompt (i.e., manual intervention).

### System Design and Illustrative Embodiments

Figure 4 shows a schematic representation of the design of the inventive portable automated single-use DFVF filter cart system that can operate either a depth filter (426a), in a so-called "Stage 1" mode or position, or a viral filter (426b) in a so-called "Stage 2" mode or position, in either batch or continuous mode. As further illustrated in Figure 4, the system design can accommodate a variety of prefilter, sterilizing grade filter (426c), and/or depth filter (426a), and/or viral filter (426b) configurations, either located on the filter cart (see e.g., in Figure 1 at 126b), or, optionally, off the perimeter of the filter cart (denoted as "P" in Figure 4). These filters can be installed in 3 different positions, also referred to as "Stages," on either the first or second filtration path. "Stage 1" refers to the first position (426a) in the filter path, "Stage 2" refers to the second position (426b) in the filter path, and "Stage 3" refers to the third position in the filter path. For example, in some embodiments a depth filtration process can position a depth filter (426a) at Stage 1, located outside the perimeter ("P) of the filter cart system (see, also Figure 3) and, optionally, a sterilizing grade filter (426c) can be positioned at Stage 3 on the filter cart (i.e., within "P"; see, e.g., in Figure 1 at 126c), as schematically shown at 426c. In this example, the Stage 2 position (426b) is bypassed by using a piece of tubing to connect the rest of the tubing manifold sections instead of a filter to complete the flow path. In an example viral filtration process, a prefilter can be located at Stage 1 on the filter cart (i.e., within the cart perimeter "P") as denoted by 426a, a viral filter (426b) positioned at Stage 2 either on the filter cart (i.e., within "P") or outside the perimeter (426b), and a sterilizing grade filter at Stage 3 on the filter cart system (426c; within "P"). The filters can be positioned in either the first filtration path or the second filtration path, or both, in the case of a continuous manufacturing process, with filter swapping.

The system also includes a set of inlets (401, 402), including (i) one or more valved buffer inlets (402) and (ii) a product inlet (401) for receiving an aqueous product-containing fluid from an upstream unit operation fluidly connected thereto upstream from the first filtration path and the second filtration path. The inlets (401, 402) are fluidly connected via tubing to a set of inlet valves (410a, 410b) and a set of pumps (416a, 416b) to perform buffer flushing or product filtration operations.

The first pump (416a) is designed for low flow rate operation in a continuous manufacturing process, and is used to load product-containing fluid from the product inlet (401) through the product inlet valve (410a) onto the filters during a continuous process. A second pump (416b) is designed for high flow rate operation, and is used to perform buffer flushing, equilibration, or sanitization operations for either a batch or continuous process; the second pump (416b) is also used to load product onto the filters (426a, 426b, 426c) during a batch process. The system also includes a set of inlets (401, 402), including (i) one or more valved buffer inlets (402) and (ii) a product inlet (401) for receiving an aqueous product-containing fluid from an upstream unit operation fluidly connected thereto upstream from the first filtration path and the second filtration path. The product-containing fluid is fluidly conveyed to the product inlet (401), or the buffer(s) used are fluidly conveyed to the one or more buffer inlets (402), by tubing. The inlets (401, 402) are also fluidly connected via tubing to a set of inlet valves (410a, 410b) and a set of pumps (416a, 416b) to perform buffer flushing or product filtration operations. The one or more inlets (402), and selection of which buffer inlet (402) will be operated is automatically controlled by the buffer inlet valves (410b); the automation that controls the product inlet valve (410a) and the buffer inlet valves (410b) is under the direction of the controller (440). In the schematic illustration of Figure 4, the controller (440) is shown being outside the perimeter ("P") of the cart, but this is merely for convenience and drawing clarity, and the controller can be within the perimeter of the cart (see, e.g., Figure 1 at (140) and Figure 2 at (240)) or outside the perimeter of the cart, so long as measurement and/or instruction signals, as the case may be, can be readily exchanged between the analytical sensors (418, 420), valves (410), pumps (416a, 416b), and the controller (440). The pumps (416a, 416b) can be operated in either (i) flow-control mode using the flowmeters (414), which are configured within both the first filtration path and the second filtration path, or (ii) in pressure-control mode using the one or more pressure sensors (418), which are configured at intervals within both the first filtration path and the second filtration path. For example, in Figure 4, the pressure sensors (418) are located before the first filter position (i.e., before the DF or "Stage 1"; 426a), between the first (DF) position (426a) and the second filter (VF; 426b) position (i.e., "Stage 2"), and between the second filter (VF; 426b) and third filter (i.e., "Stage 3" or sterilizing grade filter; 426c) position, in both the first filtration path and the second filtration path.

In flow-control mode, the automation adjusts the pump output (416a or 416b) based on a feedback loop from the measurement value on its corresponding flowmeter (414) in order to achieve a setpoint value predetermined and configured in the automation. In pressure-control mode, the automation adjusts the pump output (416a or 416b) based on a feedback loop from the measurement value on the pressure sensors (418); in this example, the pressure differential across the viral filter (426b) uses the pressure sensor located before and after the second filter position, and is used to achieve a setpoint value predetermined and configured in the automation.

For a batch manufacturing process, Figure 4 shows the "crossover" valves (410c, 410d, 410e, 410f), which can be automatically positioned to direct product or buffer flow from the high flow pump (416b) to the first filtration path (i.e., open 410e, close 410f and 410c), or to the second filtration path (i.e., open 410f, close 410d and 410e). For a continuous manufacturing process, the crossover valves can be automatically positioned to direct product from the low flow pump (416a) to the first filtration path (i.e., open 410c, close 410d and 410e) or to the second filtration filter path (i.e., close 410c and 410f, open 410d). For buffer flushing in a continuous process, the crossover valves can be automatically positioned to direct buffer flow from the high flow pump (416b) to the first filtration path (i.e., open 410e, close 410f and 410c), or to the second filtration path (i.e., open 410f, close 410d and 410e). In a continuous process, buffer flushing via the high flow pump (416b) can be performed simultaneously with product filtration via the low flow pump (416a).

During buffer flushing operation, the buffer can be flushed to waste for the Stage 1 or Stage 2 filter using waste outlet valves (410g), which are configured on both the first filtration path and the second filtration path. During the initiation of the product filtration, the residual volume in the first and second filtration paths can be flushed to waste by flowing through valves (410i), (410g), and/or (410j) to a waste port (waste ports are not illustrated in Figure 4, but see, e.g., Figure 1, at (104) and Figure 2, at (204)). When product is switched to collection of product through the product outlet port (103, 203, 403) to the downstream unit operation, one of the product outlet valves (410k) can be opened to the product outlet port (403). A manual valve (412) is used to introduce air to the viral filter (426b) for pressure hold or air diffusion integrity tests.

Analytical sensors (420a, 420b, 420c, 420d) are positioned at, or near, the product outlet port (403) of the filter cart system to monitor UV (420a), conductivity (420b, 420c), and pH (420d). The sensors can be used in automation to transition the start or end of buffer flushes or product collection.

Figure 1 and Figure 2 show an embodiment of the portable automated single-use filter cart system from differing perspectives. Portability is optionally enhanced by wheels (130, 230) and a handle (150, 250) for manipulating the position of the filter cart. One or more other optional buffer inlets (shown in Figure 1 as 102, but not shown in the perspective of Figure 2) are present on the filter cart for the introduction of buffer(s) or water in the first filtration path or into the second filtration path to perform buffer flushing or product filtration operations. In this embodiment, each of the first filtration path and the second filtration path includes a pre-filter (e.g., 126a, 226a), or a depth filter, or a viral filter (e.g., 126b, 226b), and/or a sterilizing grade filter (126c, 226c). In the perspectives of Figure 1 and Figure 2, the first filtration path is shown as the upper path farther from the viewer, and the second filtration path is shown as the lower path closer to the viewer, on the top of the cart (which is within the perimeter of the cart). The system employs automated valves (110, 210), controlled by a controller (140, 240), to direct the flow of an aqueous product-containing fluid, which is conveyed through a connecting line of tubing; initially flow is from an upstream unit operation into the system via a product inlet (101, 201) through the automated product inlet valve (110a), while, optionally, the one or more buffer inlets (102; not shown in the perspective of Figure 2) convey buffer(s) or water into the system via the automated buffer inlet valve(s) (110b; not shown in the perspective of Figure 2). The automatic valves (110, 210) in this embodiment and many other embodiments of the invention are pneumatic valves, robotically actuated by compressed air conveyed by a separate air plumbing system within the cart. Any suitable source of compressed air can be employed. Figure 1 and Figure 2 illustrate the air intake (161, 261) for the compressed air plumbing system, and the perspective of Figure 2 shows an air regulator (260) for the air plumbing system. A manual valve (112, 212), in each of the first and second filtration pathways, is used to introduce air to the viral filter (126b, 226b) for pressure hold or air diffusion integrity tests. Flow of the aqueous product-containing fluid is impelled by either one of two pumps; the first pump (116a, 216a) is designed for low flow rate operation in a continuous manufacturing process, and this pump (116a, 216a) is used to load product from the product inlet (101) through the automated product inlet valve (110a; not shown in the perspective of Figure 2) onto the filters during a continuous process. A second pump (shown in Figure 1 as 116b, but not shown in the perspective of Figure 2) is designed for high flow rate operation, and is used to perform buffer flushing, equilibration, or sanitization operations for either a batch or continuous process; the second pump (116b) is also used to load product onto the filters during a batch process. Flowmeters (shown in Figure 1 as 114, but not shown in the perspective of Figure 2) measure the flow rate and provide input of these data to the controller (140, 240). Pressure transducers (118, 218) at various locations in the system convert pressure into an analog electrical signal that is input to the controller (140, 240). Other analytical sensors (120, 220) are positioned at or near the product outlet port (203; product outlet port is not shown in the perspective of Figure 1) of the filter cart system, to monitor UV absorbance, conductivity, temperature, and pH, which information is also input to the controller (140, 240).

In Figure 3, another different embodiment of the portable automated single-use filter cart is illustrated with depth filters (326a) outside of the perimeter of the cart in two portable accessory modules (with optional filter housings or holders holding the filters, as shown in the embodiment in Figure 3), one depth filter for each of the first filtration path and the second filtration path. In this embodiment portability is optionally enhanced by the presence of wheels (330a) on both the filter cart itself and by the wheels (330b) of the accessory modules. Each of the two accessory modules shown in Figure 3 has an inlet (334) for the inflow of aqueous product-containing fluid and an outlet for post-filtration outflow (332) back into the perimeter of the filter cart.

In the embodiment shown in Figure 3, flow of the aqueous product-containing fluid is impelled by either one of two pumps; the first pump (316a) is designed for low flow rate operation in a continuous manufacturing process, and this pump (316a) is used to load product-containing fluid into the system from the product inlet (301) through the automated product inlet valve (310a) onto the depth filters (326a), and in this embodiment, sterilizing grade filters (326c), during a continuous process. Other embodiments can also, optionally, have viral filtration capability installed in the first and second filtration pathways, as in the embodiment shown in Figure 1 and Figure 2 (126b, 226b). In the embodiment illustrated in Figure 3, a second pump (316b) is designed for high flow rate operation, and is used to perform buffer flushing, equilibration, or sanitization operations for either a batch or continuous process; the second pump (316b) is also used to load product-containing fluid onto the filters during a batch process. Flowmeters (314) measure the flow rate, and other analytical sensors (320), optionally at or near the product outlet port (outlet port is not shown in the perspective of Figure 3), can provide input of data (e.g., about UV absorbance, conductivity, temperature, and pH) to the controller (not shown in the perspective of Figure 3). Optionally, one or more pressure sensors (318) can be configured at intervals within both the first filtration path and the second filtration path.

For a batch manufacturing process, the embodiment shown in Figure 3 includes "crossover" valves (310c, 310d, 310e, 310f), which can be automatically switched by instruction from the digital controller to direct the flow of product-containing fluid or buffer impelled by the high flow pump (316b) to the first filtration path (i.e., open 310e, close 310f and 310c), or to the second filtration path (i.e., open 310f, close 310d and 310e). For a continuous manufacturing process, the crossover valves can be automatically switched to direct the flow of product-containing fluid impelled by the low flow pump (316a) to the first filtration path (i.e., open 310c, close 310d and 310e) or to the second filtration path (i.e., close 310c and 310f, open 310d). For buffer flushing in a continuous process, the crossover valves can be automatically positioned to direct buffer flow from the high flow pump (316b) to the first filtration path (i.e., open 310e, close 310f and 310c), or to the second filtration path (i.e., open 310f, close 310d and 310e). In a continuous process, buffer flushing via the high flow pump (316b) can be performed simultaneously with product-containing fluid filtration flow impelled by the low flow pump (316a). The optional one or more buffer inlets (302), and selection of which buffer inlet (302) will be operated is automatically controlled by the buffer inlet valve(s) (310b); the automation that controls the product inlet valve (310a) and the buffer inlet valves (310b) is under the direction of the controller (not shown in the perspective of Figure 3). In the embodiment shown in Figure 3, the pumps (316a, 316b) are operated in flow-control mode using the flowmeters (314), which are configured within both the first filtration path and the second filtration path.

The following Table 1 shows some useful examples of commercially available mechanical components that can be employed in constructing the inventive filter cart, but the examples in Table 1 are merely illustrative and not an exhaustive list of such useful components.

**Table 1. Illustrative examples of commercially available mechanical components for incorporation into the inventive filter cart system.**

| Component | Manufacturer | Part number | Description |
|---|---|---|---|
| Valves | Acro | Model 933, 934, 935 | Two-way pneumatic pinch valve |
| | Fluid line | SU2FM001M0M | Manual pinch valve with clampon tubing cover |
| Pumps | Quattroflow | QF1200SU-HT, QF12DISPP-3-EZ, QF150SU, QF30SU | Quaternary diaphragm pump with single-use head |
| Flow sensors | Sonotec | CO.55/080SD, 160SD | |
| Analytical sensors | Optek-Danulat | AF46, ACF60-35, SUC07 | UV, conductivity, pH with single-use flow cell |
| Pressure sensors | Pendotech | PMAT3A, PRESS-N-038 | Single-use pressure sensor |
| Filters and filter holders | Millipore | KHGES1TTT1 | Opticap^{®} XLT 10 SHC |
| | Asahi Kasei | 20F1-000 | Planova^{®} 20N |
| | Millipore | MA1HC, MX0SP | Millistak+^{®} depth filters and filter holders |

By way of further illustration, the following numbered embodiments are encompassed by the present invention:
Embodiment 1: A portable automated single-use filter cart system for interchangeable continuous or batch biologics manufacturing, comprising:
   (a) a portable cart having a perimeter;
   (b) a valved product inlet within the perimeter of the cart for receiving an aqueous product-containing fluid from an upstream unit operation outside the perimeter of the portable cart, wherein the upstream unit operation can be fluidly connected to the product inlet by a sealable connector and a connecting line, wherein the product inlet is connected via a connecting line capable of fluidly conveying the aqueous product-containing fluid downstream to a first pump or, interchangeably, to a second pump, wherein the first pump is adapted for low flow rate operation, and the second pump is adapted for high flow rate operation, and wherein the first pump and the second pump are each configured to impel the flow of the aqueous product-containing fluid to a first filtration path or alternately to a second filtration path;
      wherein:
      the first filtration path and the second filtration path each comprises:
      (i) a plurality of filters downstream from the first pump, wherein the plurality of filters is fluidly connected in sequence along the filtration path; and, optionally, outside the perimeter of the cart, one or more portable accessory filtration module(s) fluidly connected to the filtration path and capable of receiving aqueous product-containing fluid therefrom, and capable of returning filtered aqueous product-containing fluid thereto;
      (ii) a plurality of sensors capable of continuously measuring flow rate, flow volume, and/or pressure at one or more locations along the filtration path and transmitting measurement signals to a controller;
         and
      (iii) a connecting line capable of fluidly conveying filtered aqueous product-containing fluid to a downstream product outlet port;
   (c) one or more valved buffer inlets within the perimeter of the cart and upstream from the first filtration path and the second filtration path, and each buffer inlet fluidly connected by a connecting line capable of fluidly conveying a desired aqueous fluid received from a reservoir or an upstream unit operation, for injection into the first or into the second filtration path by operation of the first pump or the second pump and the valved buffer inlets, in response to instruction signals from the controller; and
      wherein
   (d) the controller is configured and programmable to automatically switch the flow of product-containing fluid from the first filtration path to the second filtration path in response to a predetermined measurement signal received from the sensor(s) of the first filtration path, or after a predetermined period, wherein the controller is further configured and programmable to automatically switch the flow of aqueous product-containing fluid from the second filtration path to the first filtration path in response to a predetermined measurement signal received from the sensor(s) of the second filtration path, and wherein the controller is further configured and programmable to issue instruction signals:
      (i) activating the first pump during a desired continuous biologics manufacturing process, or interchangeably, activating the second pump during a desired batch biologics manufacturing process;
      (ii) activating the second pump during the desired continuous biologics manufacturing process to flush buffer or water through the first filtration pathway while the flow of aqueous product-containing fluid is through the second filtration pathway, or interchangeably, to flush buffer or water through the second filtration pathway while the flow of aqueous product-containing fluid is through the first filtration pathway; and
      (iii) controlling the valved product inlet and the one or more valved buffer inlets.
Embodiment 2: The portable automated single-use filter cart system of Embodiment 1, wherein the controller is configured and programmable for a continuous manufacturing process.
Embodiment 3: The portable automated single-use filter cart system according to any of Embodiments 1-2, wherein the controller is configured and programmable for a batch manufacturing process.
Embodiment 4: The portable automated single-use filter cart system according to any of Embodiments 1-3, wherein the controller is configured and programmable to interact with a production facility control system.
Embodiment 5: The portable automated single-use filter cart system according to any of Embodiments 1-4, comprising a depth filter.
Embodiment 6: The portable automated single-use filter cart system according to any of Embodiments 1-5, comprising a viral filter.
Embodiment 7: The portable automated single-use filter cart system according to any of Embodiments 1-6, comprising a sterilizing grade filter.
Embodiment 8: The portable automated single-use filter cart system according to any of Embodiments 1-7, comprising the one or more portable accessory filtration modules.

The following working examples are illustrative and not to be construed in any way as limiting the scope of the invention.

### EXAMPLES

### Example 1. Depth Filtration in a Continuous Process

### Materials and Methods.

Process setup. A continuous end-to-end (E2E) process was set up from SUB/Perfusion to continuous capture, including continuous two-tank viral inactivation, followed by continuous depth filtration. A surge vessel was used between each unit operation to manage flow discrepancies between the process steps. Monoclonal antibody (mAb) product was perfused through a 0.2-µm filter as previously described in Vandiver et al., Automated Biomanufacturing Systems, Facilities, and Processes, WO2020/168315A1. Harvest collection started on production day 8 with downstream steps phasing in as surge vessels met their operating volume.

A X0SP 0.55m² depth filter (Millistak+^{®}; Millipore Sigma) was used with a SHC 0.2µm sterilizing grade filter (Millipore Express^{®}; Millipore Sigma) to remove turbidity and process related impurities. Product loading was set to 6 days of operation, or until a maximum pressure of 20 psi was reached. Loading can be set by volume or time, in this example time-based switching was used. Production ended on day 22 and downstream operations were phased out as surge vessel met minimum volume.

Installation. A DF/VF tubing manifold (i.e. the tubing system) was installed as shown in Figures 1-3, including probes, sensors, single-use pump heads with AseptiQuik^{®} fittings (CPC, Colder Products Company) to connect different parts of the tubing manifold together in an aseptic manner. Buffer connecting lines were attached to inlets aseptically. The product load inlet was connected to the preceding feed surge vessel. Outlet waste streams were connected together to one waste outlet, connected to drain by a single-use air break assembly, in order to maintain a closed system. (See, Vandiver et al., Automated Biomanufacturing Systems, Facilities, and Processes, WO2020/168315A1). Product outlet port was connected to a collection surge vessel. A depth filter was installed into the POD holder (Catalog No. MP0DPIL0T, Millipore Sigma) using silicone tubing connecting lines coming off the filter cart in the "Stage 1" (i.e., DF) position to the POD inlet, and back from the POD outlet to return flow back to the cart. A 0.2-µm filter was inserted in the "Stage 3" (i.e., sterilizing grade filter) position and connected to the tubing manifold aseptically.

Flushing. The following steps were performed using an automated sequence in a DeltaV Distributed Control System (DCS) automation system (Emerson):
Filter Path 1 (i.e., first filtration path, arbitrarily represented as the upper path in Figure 4):
Prime inlet connecting lines and depth filter to purge air from the system;
Flush the depth filter with water per manufacturer's recommendation to the "Stage 1" waste outlet valve (410g in Figure 4); then to the final waste outlet valve (410j in Figure 4), and thence to the waste outlet connected thereto;
Flush with 0.5N NaOH to the "Stage 1" waste outlet and hold for 30 min to sanitize the filter and POD holder connections;
Flush out NaOH with equilibration buffer (EQ), first to the "Stage 1" waste outlet valve (410g); then to the final waste outlet valve (410j in Figure 4), and thence to the waste outlet connected thereto;
Allow automation to initiate loading on the depth filter once the feed surge vessel reaches the start weight. Depth filter load flowrate was based on the continuous process flow to maintain level control in the surge vessel.

The automation was allowed to change the product load flowrate setpoint to fast, normal, or slow, depending on the tank level in the preceding surge vessel, in order to control the volume in the surge vessel to a predetermined range. The initial buffer was held up in the connecting lines and filter was flushed to drain through a waste outlet valve (410j in Figure 4), then switched to the product collection outlet valve (410k in Figure 4), and thence to the product outlet port (403). The first filter was loaded for 6 days, and the automation monitored the system for pressure, while continuously controlling the flowrate.

Filter swap. Prior to the end of Filter Path 1 loading, the Filter Path 2 (i.e., second filtration path, arbitrarily represented as the lower path in Figure 4) depth filter was prepared in a similar manner with all of the pre-use flushes occurring on the filter cart system driven by the automation. The flushing of Filter Path 2 was performed simultaneously with product loading on Filter Path 1, using the valving configurations shown in Figure 4. When Filter Path 1 had 10% of load time remaining, the automation triggered a flag to initiate the final EQ flush step of Filter Path 2. When Filter Path 1 reached the end of the load time, the automation switched the product load flow path from Filter Path 1 loading to Filter Path 2 loading, directed to the waste outlet valve on Filter Path 2 (410j, lower path in Figure 4) and thence to the waste outlet connected thereto. After the switch to product loading on Filter Path 2 occurred, the automation initiated EQ recovery flush from the high flow pump (416b) through Filter Path 1 to the product collection outlet valve on Filter Path 1 (410k, upper path in Figure 4), and thence to the product outlet port (403), to recover product held up in the filter and tubing connecting lines. The EQ recovery flush on Filter Path 1 was performed simultaneously with product loading on Filter Path 2. After reaching the pre-determined volume transition point, the automation switched the Filter Path 2 from the waste valve (410j, lower path in Figure 4) to product collection (410k, lower path in Figure 4) and the Filter Path 1 operation was completed. The same procedure for filter switching to the third set of depth filters occurred in the same manner as described above, with the new set of filters prepared on Filter Path 1 and switching back to product loading on Filter Path 1 once the second depth filter loading was completed. A total of 3 depth filters were used for this process for this experiment, however, the alternation between Filter Path 1 and Filter Path 2 can continue for as many times as required for a given continuous process, if desired.

### Results.

Depth Filter (DF) flushing performance. A flow rate of 2.3 liters per minute (L/min) was used for all flush steps. The pre-depth filter pressure started at 10 psi and slowly decreased to 8-9 psi during the flushes. At the end of the analytical flush, the pH was slightly different (0.1-0.3 pH units higher than EQ buffer) between the filter paths and to the EQ buffer, which could have been due to an offset with the pH probe following autoclaving. It is a standard procedure to perform a one-point calibration adjustment on the system pH transmitter to align the pH of the probe with an offline reading, but this remedy was not employed for this run. Table 2 summarizes the flushing steps, and the time and volume it took to perform each step. Including transitions, the priming and flushing steps took about 2 hours.

**Table 2. DF flushing performance. *Analytical flush was performed with EQ buffer flushing the entire tubing flow path out the post-"Stage 3" (sterilizing grade filtration) waste valve (410j in Figure 4) to waste port.**

| Step | Buffer | Time (min) | Volume (L) |
|---|---|---|---|
| Water for injection (WFI) Flush | WFI | 24 | 55 |
| Sanitization Flush | 0.5N NaOH | 7 | 14 |
| Sanitization Hold | NA | 30 | NA |
| Equilibration | Sodium Phosphate | 37 | 83 |
| Analytical Flush* | Sodium Phosphate | 6 | 12 |

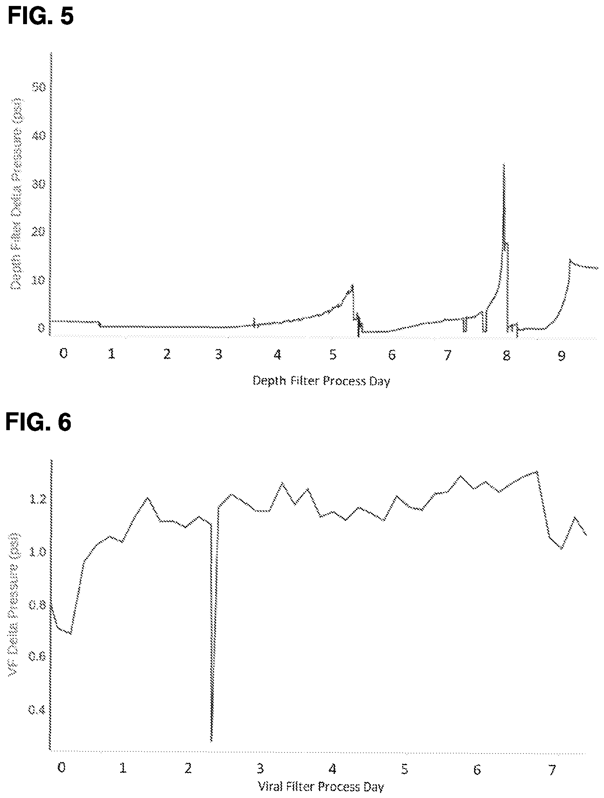

The first depth filter was loaded with flow over a period of 6 days. As shown in Figure 5, a pressure increase was observed for each of the filters, and the filter swap occurred earlier than 6 days for Filters 2 and 3, due to an increase in turbidity of the load. The pressure increase and duration of loading can be modified as desired in future runs by increasing the filter area used in the process.

### Example 2. Viral Filtration in a Continuous Process

### Materials and Methods.

Process setup. From the DF process as described in Example 1 (above), the process went through two polishing chromatography steps into a surge vessel preceding the viral filtration step. The product was titrated with acid buffer in the preceding load surge vessel. Harvest collection started on production day 8 with downstream steps phasing in as surge vessels met their operating volumes. A Planova^{™} 20N virus removal filter (1m² ; Asahi Kasei), sized to 1000 L/m², was employed, without swapping during this process run. Production ended on day 22 and downstream operations were phased out as surge vessel met minimum volume.

Installation. A tubing manifold was installed, as described in Example 1 (above), including probes, sensors, single-use pump heads, with AseptiQuik^{®} fittings to connect different parts of the tubing manifold together in aseptic manner. Buffer connecting lines were attached to inlets aseptically. The product load inlet was connected to the preceding feed surge vessel. Outlet waste streams were connected together to one waste outlet, which was connected to drain by a single-use air break assembly, in order to maintain a closed system. (See, Vandiver et al., Automated Biomanufacturing Systems, Facilities, and Processes, WO2020/168315A1). The product outlet port was connected to the collection surge vessel. The viral filter was installed into the "Stage 2" (VF) filter holder and was connected aseptically to the inlet (feed) and outlet (permeate) tubing manifolds. The retentate was aseptically connected to tubing that was manually clamped. A 0.2-µm filter was inserted in the "Stage 1" (DF) position as a prefilter and connected to the tubing manifold aseptically.

Pre-use leakage test. A pre-use leakage test was not performed for this demonstration run, but the inventive system includes a dedicated valve to attach air to pressurize the filter to the required amount and perform a pressure hold test per manufacturer procedure.

Flushing. The following steps were performed using an automated sequence in a DeltaV Distributed Control System (DCS) automation system (Emerson):
Filter Path 1 (i.e., first filtration path, arbitrarily represented as the lower path in Figure 4):
Prime inlet connecting lines and filter to purge air from the system;
Flush the viral filter with water per manufacturer's recommendation, including purging air via the retentate port; flushing through the viral filter permeate was to the "Stage 2" (VF) waste outlet valve (410g, upper path in Figure 4) and thence to the waste outlet;
Flush equilibration buffer (EQ) buffer through the permeate to the "Stage 2" waste outlet valve (410g, upper path in Figure 4) and thence to the waste outlet connected thereto;
Allow automation to initiate loading on the viral filter once the feed surge vessel reaches the start weight and the feed tank reaches pH setpoint.

The pH titration was performed in the feed surge vessel with acid buffer attached to a titrant pump. Automation was used to achieve the pH setpoint. The automation paused the viral filter loading, if the pH went out of process specification, and resumed once the pH came back within range. The viral filter load flowrate was based on the continuous process flow to maintain level control in the surge vessel. The automation was allowed to change the product load flowrate setpoint to fast, normal, or slow, depending on the tank level in the preceding surge vessel, in order to control the volume in the surge vessel to a predetermined range.

The initial buffer was held up in the connecting lines, and the filter was flushed to drain, then flow was switched to the product outlet port. The automation monitored the system for pressure, and alarms were enabled to alert the operator to high pressure or flow deviations, if they occurred.

As described in Example 1, the viral filter can be swapped between Filter Path 1 (i.e., first filtration path, arbitrarily represented as the upper path in Figure 4) to Filter Path 2 (i.e., second filtration path, arbitrarily represented as the lower path in Figure 4) and back to Filter Path 1, however, for this demonstration process, only one viral filter was used for the duration of the run, and no filter swapping was performed, although filter swapping can be done for a longer run.

Results. Viral filter was operated for 8 days of continuous processing. During this time, the inlet pressure of the Planova^{™} filter showed minimal increase from 0.7 to 1.3 psi (Figure 6).

## Claims

1. A portable automated single-use filter cart system for interchangeable continuous or batch biologics manufacturing, comprising:
(a) a portable cart having a perimeter (P);
(b) a valved product inlet (101, 201, 301, 401) within the perimeter (P) of the cart for receiving an aqueous product-containing fluid from an upstream unit operation outside the perimeter (P) of the portable cart, wherein the upstream unit operation can be fluidly connected to the product inlet (101, 201, 301, 401) by a sealable connector and a connecting line, wherein the product inlet (101, 201, 301, 401) is connected via a connecting line capable of fluidly conveying the aqueous product-containing fluid downstream to a first pump (116a, 216a, 316a, 416a) or, interchangeably, to a second pump (116b, 316b, 416b), wherein the first pump is adapted for low flow rate operation, and the second pump is adapted for high flow rate operation, and wherein the first pump and the second pump are each configured to impel the flow of the aqueous product-containing fluid to a first filtration path or alternately to a second filtration path;
wherein:
the first filtration path and the second filtration path each comprises:
(i) a plurality of filters (426a, 426b, 426c) downstream from the first pump, wherein the plurality of filters is fluidly connected in sequence along the filtration path; and, optionally, outside the perimeter of the cart, one or more portable accessory filtration module(s) fluidly connected to the filtration path and capable of receiving aqueous product-containing fluid therefrom, and capable of returning filtered aqueous product-containing fluid thereto;
(ii) a plurality of sensors (118, 218, 318, 418, 114, 314, 414, 120, 220, 320, 420, 420a, 420b, 420c, 420d) capable of continuously measuring flow rate, flow volume, and/or pressure at one or more locations along the filtration path and transmitting measurement signals to a controller (140, 240, 440);
and
(iii) a connecting line capable of fluidly conveying filtered aqueous product-containing fluid to a downstream product outlet port (103, 203, 403);
(c) one or more valved buffer inlets (102, 302, 402) within the perimeter of the cart and upstream from the first filtration path and the second filtration path, and each buffer inlet fluidly connected by a connecting line capable of fluidly conveying a desired aqueous fluid received from a reservoir or an upstream unit operation, for injection into the first or into the second filtration path by operation of the first pump (116a, 216a, 316a, 416a) or the second pump (116b, 316b, 416b) and the valved buffer inlets (102, 302, 402), in response to instruction signals from the controller (140, 240, 440); and
wherein
(d) the controller (140, 240, 440) is configured and programmable to automatically switch the flow of product-containing fluid from the first filtration path to the second filtration path in response to a predetermined measurement signal received from the sensor(s) (118, 218, 318, 418, 114, 314, 414, 120, 220, 320, 420, 420a, 420b, 420c, 420d) of the first filtration path, or after a predetermined period, wherein the controller is further configured and programmable to automatically switch the flow of aqueous product-containing fluid from the second filtration path to the first filtration path in response to a predetermined measurement signal received from the sensor(s) of the second filtration path, and wherein the controller is further configured and programmable to issue instruction signals:
(i) activating the first pump (116a, 216a, 316a, 416a) during a desired continuous biologics manufacturing process, or interchangeably, activating the second pump (116b, 316b, 416b) during a desired batch biologics manufacturing process;
(ii) activating the second pump (116b, 316b, 416b) during the desired continuous biologics manufacturing process to flush buffer or water through the first filtration pathway while the flow of aqueous product-containing fluid is through the second filtration pathway, or interchangeably, to flush buffer or water through the second filtration pathway while the flow of aqueous product-containing fluid is through the first filtration pathway; and
(iii) controlling the valved product inlet (101, 201, 301, 401) and the one or more valved buffer inlets (102, 302, 402).

2. The portable automated single-use filter cart system of Claim 1, wherein the controller (140, 240, 440) is configured and programmable for a continuous manufacturing process.

3. The portable automated single-use filter cart system according to any of Claims 1-2, wherein the controller (140, 240, 440) is configured and programmable for a batch manufacturing process.

4. The portable automated single-use filter cart system according to any of Claims 1-3, wherein the controller (140, 240, 440) is configured and programmable to interact with a production facility control system.

5. The portable automated single-use filter cart system according to any of Claims 1-4, comprising a depth filter (326a, 426a).

6. The portable automated single-use filter cart system according to any of Claims 1-5, comprising a viral filter (126b, 226b, 426b).

7. The portable automated single-use filter cart system according to any of Claims 1-6, comprising a sterilizing grade filter (126c, 226c, 326c, 426c).

8. The portable automated single-use filter cart system according to any of Claims 1-7, comprising the one or more portable accessory filtration modules.

## Patentansprüche

1. Transportables automatisiertes Einwegfilterwagensystem für eine austauschbare kontinuierliche oder Chargenfertigung von Biologika, umfassend:
(a) einen transportablen Wagen, der einen Umfang (P) aufweist;
(b) einen mit einem Ventil versehenen Produkteinlass (101, 201, 301, 401) innerhalb des Umfangs (P) des Wagens zum Aufnehmen eines wässrigen produkthaltigen Fluids von einer stromaufwärtigen Grundoperation außerhalb des Umfangs (P) des tragbaren Wagens, wobei die stromaufwärtige Grundoperation mittels eines abdichtbaren Verbinders und eine Verbindungsleitung mit dem Produkteinlass (101, 201, 301, 401) fluidisch verbunden werden kann, wobei der Produkteinlass (101, 201, 301, 401) über eine Verbindungsleitung verbunden ist, die zum fluidischen Leiten des wässrigen produkthaltigen Fluids stromabwärts zu einer ersten Pumpe (116a, 216a, 316a, 416a) oder, austauschbar, zu einer zweiten Pumpe (116b, 316b, 416b) in der Lage ist, wobei die erste Pumpe für eine Operation mit niedriger Durchflussmenge angepasst ist und die zweite Pumpe für eine Operation mit hoher Durchflussmenge angepasst ist und wobei die erste Pumpe und die zweite Pumpe jeweils konfiguriert sind, um den Durchfluss des wässrigen produkthaltigen Fluids zu einem ersten Filtrationsweg oder alternativ zu einem zweiten Filtrationsweg zu treiben;
wobei:
der erste Filtrationsweg und der zweite Filtrationsweg jeweils umfassen:
(i) eine Vielzahl von Filtern (426a, 426b, 426c) stromabwärts von der ersten Pumpe, wobei die Vielzahl von Filtern entlang des Filtrationswegs der Reihe nach fluidisch verbunden ist; und, optional, außerhalb des Umfangs des Wagens, ein oder mehrere transportable(s) Hilfsfiltrationsmodul(e), das/die mit dem Filtrationsweg fluidisch verbunden und zum Aufnehmen des wässrigen produkthaltigen Fluids davon in der Lage und zum Zurückführen eines gefilterten wässrigen produkthaltigen Fluids dorthin in der Lage ist/sind;
(ii) eine Vielzahl von Sensoren (118, 218, 318, 418, 114, 314, 414, 120, 220, 320, 420, 420a, 420b, 420c, 420d), die zum kontinuierlichen Messen der Durchflussmenge, eines Durchflussvolumens und/oder eines Drucks an einer oder mehreren Stellen entlang des Filtrationswegs und Übertragen von Messsignalen an eine Steuerung (140, 240, 440) in der Lage ist;
und
(iii) eine Verbindungsleitung, die zum Leiten des gefilterten wässrigen produkthaltigen Fluids zu einer stromabwärtigen Produktauslassöffnung (103, 203, 403) in der Lage ist;
(c) einen oder mehrere mit Ventilen versehene Puffereinlässe (102, 302, 402) innerhalb des Umfangs des Wagens und stromaufwärts von dem ersten Filtrationsweg und dem zweiten Filtrationsweg und wobei jeder Puffereinlass mittels einer Verbindungsleitung fluidisch verbunden ist, die zum fluidischen Leiten eines gewünschten wässrigen Fluids, das aus einem Reservoir oder einer stromaufwärtigen Grundoperation aufgenommen wird, für eine Einspritzung in den ersten oder in den zweiten Filtrationsweg mittels einer Operation der ersten Pumpe (116a, 216a, 316a, 416a) oder der zweiten Pumpe (116b, 316b, 416b) und der mit Ventilen versehenen Puffereinlässe (102, 302, 402) als Reaktion auf Anweisungssignale von der Steuerung (140, 240, 440) in der Lage ist; und
wobei
(d) die Steuerung (140, 240, 440) konfiguriert und programmierbar ist, um den Durchfluss des produkthaltigen Fluids von dem ersten Filtrationsweg zu dem zweiten Filtrationsweg als Reaktion auf ein zuvor bestimmtes Messsignal, das von dem/den Sensor(en) (118, 218, 318, 418, 114, 314, 414, 120, 220, 320, 420, 420a, 420b, 420c, 420d) des ersten Filtrationswegs empfangen wird, oder nach einer zuvor bestimmten Zeitspanne automatisch umzuschalten, wobei die Steuerung ferner konfiguriert und programmierbar ist, um den Durchfluss des wässrigen produkthaltigen Fluids von dem zweiten Filtrationsweg zu dem ersten Filtrationsweg als Reaktion auf ein zuvor bestimmtes Messsignal, das von dem/den Sensor(en) des zweiten Filtrationswegs empfangen wird, automatisch umzuschalten, und wobei die Steuerung ferner konfiguriert und programmierbar ist, um die Anweisungssignale auszugeben:
(i) Aktivieren der ersten Pumpe (116a, 216a, 316a, 416a) während eines gewünschten kontinuierlichen Fertigungsprozesses von Biologika oder, austauschbar, Aktivieren der zweiten Pumpe (116b, 316b, 416b) während eines gewünschten Chargenfertigungsprozesses von Biologika;
(ii) Aktivieren der zweiten Pumpe (116b, 316b, 416b) während des gewünschten kontinuierlichen Fertigungsprozesses von Biologika, um Puffer oder Wasser durch den ersten Filtrationspfad zu spülen, während der Durchfluss des wässrigen produkthaltigen Fluids durch den zweiten Filtrationspfad erfolgt, oder, austauschbar, um Puffer oder Wasser durch den zweiten Filtrationspfad zu spülen, während der Durchfluss des wässrigen produkthaltigen Fluids durch den ersten Filtrationspfad erfolgt; und
(iii) Steuern des mit Ventilen versehenen Produkteinlasses (101, 201, 301, 401) und des einen oder der mehreren mit Ventilen versehenen Puffereinlässe (102, 302, 402).

2. Transportables automatisiertes Einwegfilterwagensystem nach Anspruch 1, wobei die Steuerung (140, 240, 440) für einen kontinuierlichen Fertigungsprozess konfiguriert und programmierbar ist.

3. Transportables automatisiertes Einwegfilterwagensystem nach einem der Ansprüche 1 bis 2, wobei die Steuerung (140, 240, 440) für einen Chargenfertigungsprozess konfiguriert und programmierbar ist.

4. Transportables automatisiertes Einwegfilterwagensystem nach einem der Ansprüche 1 bis 3, wobei die Steuerung (140, 240, 440) konfiguriert und programmierbar ist, um mit einem Produktionsanlagensteuersystem zu interagieren.

5. Transportables automatisiertes Einwegfilterwagensystem nach einem der Ansprüche 1 bis 4, umfassend einen Tiefenfilter (326a, 426a).

6. Transportables automatisiertes Einwegfilterwagensystem nach einem der Ansprüche 1 bis 5, umfassend einen Virenfilter (126b, 226b, 426b).

7. Transportables automatisiertes Einwegfilterwagensystem nach einem der Ansprüche 1 bis 6, umfassend einen Sterilisierungsfilter (126c, 226c, 326c, 426c).

8. Transportables automatisiertes Einwegfilterwagensystem nach einem der Ansprüche 1 bis 7, umfassend das eine oder die mehreren tragbaren Hilfsfiltrationsmodule.

## Revendications

1. Système automatisé portable de chariot de filtration à usage unique pour la fabrication interchangeable de produits biologiques en continu ou par lots, comprenant :
(a) un chariot portable ayant un périmètre (P) ;
(b) une entrée de produit à valve (101, 201, 301, 401) à l'intérieur du périmètre (P) du chariot permettant de recevoir un fluide aqueux contenant un produit provenant d'une opération d'unité en amont à l'extérieur du périmètre (P) du chariot portable, dans lequel l'opération d'unité en amont peut être connectée fluidiquement à l'entrée de produit (101, 201, 301, 401) par un connecteur scellable et une conduite de raccordement, dans lequel l'entrée de produit (101, 201, 301, 401) est raccordée par le biais d'une conduite de raccordement capable d'acheminer de manière fluidique le fluide aqueux contenant un produit en aval vers une première pompe (116a, 216a, 316a, 416a) ou, de manière interchangeable, vers une seconde pompe (116b, 316b, 416b), dans lequel la première pompe est adaptée à un fonctionnement à faible débit, et la seconde pompe est adaptée à un fonctionnement à haut débit, et dans lequel la première pompe et la seconde pompe sont chacune configurées pour pousser l'écoulement du fluide aqueux contenant un produit vers une première voie de filtration ou alternativement vers une seconde voie de filtration ;
dans lequel :
la première voie de filtration et la seconde voie de filtration comprennent chacune :
(i) une pluralité de filtres (426a, 426b, 426c) en aval de la première pompe, dans lequel la pluralité de filtres est raccordée fluidiquement en séquence le long de la voie de filtration ; et, facultativement, à l'extérieur du périmètre du chariot, un ou plusieurs modules de filtration accessoires portables raccordés fluidiquement à la voie de filtration et capables de recevoir un fluide aqueux contenant un produit à partir de celle-ci, et capables de renvoyer un fluide aqueux filtré contenant un produit vers celle-ci ;
(ii) une pluralité de capteurs (118, 218, 318, 418, 114, 314, 414, 120, 220, 320, 420, 420a, 420b, 420c, 420d) capables de mesurer en continu le débit, le volume d'écoulement, et/ou la pression au niveau d'un ou plusieurs emplacements le long de la voie de filtration et de transmettre des signaux de mesure à un dispositif de commande (140, 240, 440) ;
et
(iii) une conduite de raccordement capable d'acheminer le fluide aqueux filtré contenant un produit vers un orifice de sortie de produit en aval (103, 203, 403) ;
(c) une ou plusieurs entrées tampons à valve (102, 302, 402) à l'intérieur du périmètre du chariot et en amont de la première voie de filtration et de la seconde voie de filtration, et chaque entrée tampon étant raccordée fluidiquement par une conduite de raccordement capable d'acheminer un fluide aqueux souhaité reçu d'un réservoir ou d'une opération d'unité en amont, pour l'injection dans la première ou la seconde voie de filtration par l'action de la première pompe (116a, 216a, 316a, 416a) ou de la seconde pompe (116b, 316b, 416b) et des entrées tampons à valve (102, 302, 402), en réponse à des signaux d'instruction provenant du dispositif de commande (140, 240, 440) ; et
dans lequel
(d) le dispositif de commande (140, 240, 440) est configuré et programmable pour commuter automatiquement l'écoulement de fluide contenant un produit de la première voie de filtration à la seconde voie de filtration en réponse à un signal de mesure prédéterminé reçu du ou des capteurs (118, 218, 318, 418, 114, 314, 414, 120, 220, 320, 420, 420a, 420b, 420c, 420d) de la première voie de filtration, ou après une période prédéterminée, dans lequel le dispositif de commande est en outre configuré et programmable pour commuter automatiquement l'écoulement de fluide aqueux contenant un produit de la seconde voie de filtration vers la première voie de filtration en réponse à un signal de mesure prédéterminé reçu du ou des capteurs de la seconde voie de filtration, et dans lequel le dispositif de commande est en outre configuré et programmable pour émettre des signaux d'instruction :
(i) activant la première pompe (116a, 216a, 316a, 416a) pendant un processus de fabrication de produits biologiques en continu ou, de manière interchangeable, activant la seconde pompe (116b, 316b, 416b) pendant un processus de fabrication de produits biologiques par lots ;
(ii) activant la seconde pompe (116b, 316b, 416b) pendant le processus de fabrication de produits biologiques en continu souhaité pour rincer avec un tampon ou de l'eau la première voie de filtration pendant que l'écoulement de fluide aqueux contenant un produit passe par la seconde voie de filtration, ou de manière interchangeable, pour rincer avec un tampon ou de l'eau la seconde voie de filtration pendant que l'écoulement de fluide aqueux contenant un produit passe par la première voie de filtration ; et
(iii) commander l'entrée de produit à valve (101, 201, 301, 401) et la ou les entrées tampons à valve (102, 302, 402).

2. Système automatisé portable de chariot de filtration à usage unique selon la revendication 1, dans lequel le dispositif de commande (140, 240, 440) est configuré et programmable pour un processus de fabrication en continu.

3. Système automatisé portable de chariot de filtration à usage unique selon l'une quelconque des revendications 1 à 2, dans lequel le dispositif de commande (140, 240, 440) est configuré et programmable pour un processus de fabrication par lots.

4. Système automatisé portable de chariot de filtration à usage unique selon l'une quelconque des revendications 1 à 3, dans lequel le dispositif de commande (140, 240, 440) est configuré et programmable pour interagir avec un système de commande d'installation de production.

5. Système automatisé portable de chariot de filtration à usage unique selon l'une quelconque des revendications 1 à 4, comprenant un filtre en profondeur (326a, 426a).

6. Système automatisé portable de chariot de filtration à usage unique selon l'une quelconque des revendications 1 à 5, comprenant un filtre à virus (126b, 226b, 426b).

7. Système automatisé portable de chariot de filtration à usage unique selon l'une quelconque des revendications 1 à 6, comprenant un filtre de qualité pour stérilisation (126c, 226c, 326c, 426c).

8. Système automatisé portable de chariot de filtration à usage unique selon l'une quelconque des revendications 1 à 7, comprenant le ou les modules de filtration accessoires portables.
